# EUROPÄISCHE PATENTSCHRIFT

(11) **EP 3 539 589 B1**
(45) Veröffentlichungstag und Bekanntmachung des Hinweises auf die Patenterteilung: **26.04.2023**
(21) Anmeldenummer: 19162819.7
(22) Anmeldetag: 14.03.2019
(51) Int. Cl.: A61M 1/36

(54) **SICHERUNGSEINRICHTUNG MIT NADELDISLOKATION-ERFASSUNGS- UND ABSPERRMECHANISMUS ZUM NOT-ABSPERREN EINES EXTRAKORPORALEN BLUTLEITUNGSSYSTEMS**
SAFETY DEVICE WITH NEEDLE DISLOCATION DETECTION AND SHUT-OFF MECHANISM FOR AN EMERGENCY STOP OF EXTRACORPOREAL BLOOD TRANSPORT SYSTEM
DISPOSITIF DE SÉCURITÉ À MÉCANISME DE DÉTECTION ET DE BLOCAGE DE DISLOCATION D'AIGUILLE DESTINÉ AU BLOCAGE D'URGENCE D'UN SYSTÈME EXTRACORPOREL DE CONDUITS SANGUINS

(30) Priorität: 15.03.2018 DE 102018106094
(43) Veröffentlichungstag der Anmeldung: 18.09.2019
(73) Patentinhaber: B. Braun Avitum AG, 34212 Melsungen (DE)
(72) Erfinder: RITTER, Kai-Uwe, 34212 Melsungen (DE)
(74) Vertreter: Winter, Brandl - Partnerschaft mbB

(56) Entgegenhaltungen:
- WO-A1-2009/042261
- WO-A1-2014/049357
- JP-A- 2016 214 293
- US-A1- 2006 130 591

## Beschreibung

Bei der vorliegenden Erfindung handelt es sich um eine Absperrvorrichtung einer extrakorporalen Blutbehandlungsmaschine, insbesondere Dialysemaschine, welche abhängig von einer Relativ-Bewegung zweier Patientenzugänge, insbesondere Zugangskanülen, aus einer vorbestimmten Ausgangs-Relativposition heraus einen Absperrmechanismus betätigt oder auslöst, um einen Blutfluss durch eine (Blut-) Leitung der extrakorporalen Blutbehandlungsmaschine abzusperren oder zu reduzieren. In anderen Worten ausgedrückt betrifft die vorliegende Erfindung ein extrakorporales Blutleitungssystem für eine/einer extrakorporale(n) Blutbehandlungsmaschine (Dialysemaschine), welches ein(e) Sicherungssystem/Sicherungseinrichtung (mit einer Nadeldislokation-Erfassungseinrichtung, insbesondere Relativlage-/Positionserfassungseinrichtung, und einer Absperrvorrichtung) aufweist. Das/die Sicherungssystem/Sicherungseinrichtung spricht auf eine Änderung einer Relativlage zwischen dem venösen und dem arteriellen Patientenzugang/Anschluss des extrakorporales Blutleitungssystems (welche Blutleitungssystem-eigene Patientenanschlüsse sind) an. Bei einem bestimmten oder vorbestimmbaren (erfassten) Änderungsbetrag der Relativlage das extrakorporale Blutleitungssystem sperrt oder reduziert es/sie dessen Durchflussquerschnitt zumindest abschnittsweise (an zumindest einer Stelle).

### Hintergrund der Erfindung

Dialyse, insbesondere Hämodialyse, Hämofiltration und Hämodiafiltration, stellt die häufigste Behandlungsform bei Nierenversagen dar und erfordert häufige und regelmäßige Behandlungen für einen Patienten, welche sowohl in einer Klinik durchgeführt werden können, als auch zu Hause von dem Patienten selbst.

Bei einer Dialysebehandlung werden mittels einer arteriellen Zugangskanüle (arterieller Anschluss des extrakorporalen Blutleitungssystems) ein arterieller Zugang und mittels einer venösen Zugangskanüle (venöser Anschluss des extrakorporalen Blutleitungssystems) ein venöser Zugang gelegt, um den Patienten an eine Dialysemaschine anzuschließen. Hierfür werden Venenverweilkanülen oder -katheter eingesetzt, bei welchen ein Kunststoffschlauch oder Verweilschlauch über eine Metallkanüle/Einstecknadel in eine Patientenvene eingeführt wird und nach Entfernen der Metallkanüle/Einstecknadel als (Patienten-) Zugang in der Patientenvene verbleibt. Derartige Venenverweilkanülen sind aus dem Stand der Technik hinreichend bekannt.

Wenn bei einer Dialysebehandlung sich der venöse Patientenzugang, oder genauer der in der Vene verbleibende Kunststoffschlauch, löst und teilweise oder vollständig aus dem Patientengefäß/-vene rutscht, wird dies als venöse Nadeldislokation (VND) bezeichnet. Das stellt eine kritische Komplikation und Gefahr für den Patienten dar. In einem solchen Fall fließt Blut, welches die Dialysemaschine durchflossen hat, nicht zurück in den Kreislauf des Patienten, sondern wird in die Umgebung gepumpt, insbesondere durch eine Blutpumpe der Behandlungsmaschine. Ein durchschnittlicher Patient kann bei einer VND innerhalb von 5 Minuten bis zu 50% seines Gesamtblutvolumens an Blut verlieren und es reichen bereits zwei Minuten, bis Vitalzeichen eines Patienten signifikant beeinträchtigt sind. Folglich ist es unbedingt erforderlich, eine VND möglichst schnell zu erkennen. Je nach Reaktionszeit liegen die Folgen bei einem relativ geringen Blutverlust, erhöhter Infektionsgefahr, irreversiblen Hirnschäden bis hin zum Tod. Neben den physischen und psychischen Folgen für den Patienten fallen durch benötigte Blutkonserven und Folgebehandlungen auch hohe Kosten an. Insbesondere bei Heimdialyse und nächtlichen Behandlungen, wo die Patienten zumeist schlafen, sind VND kritisch, da der Patient nicht in der Lage ist, die VND selbst zu bemerken und möglicherweise rechtzeitig zu reagieren und auf sich aufmerksam zu machen.

Eine besondere Herausforderung besteht darin, dass eine VND nahezu jederzeit auftreten kann. Zwar sind einige Risikofaktoren bekannt, wie beispielsweise Rastlosigkeit oder mentale Beeinträchtigung des Patienten sowie das Auftreten von Muskelkrämpfen während der Behandlung, jedoch treten VND auch unter Idealbedingungen auf und sind somit kaum vorhersagbar.

### Stand der Technik

Um die Gefahr einer VND zu verringern, gibt es Richtlinien zu der Art und Weise, wie ein Zugang gelegt und befestigt werden soll. Ferner existieren Lösungen, welche ein Auftreten einer VND erkennen und einen Alarm auslösen, sowie ggf. die Blutpumpe der Blutbehandlungsmaschine ausschalten können bzw. den Durchflussquerschnitt des extrakorporalen Blutleitungssystems verringern können.

Eine solche Lösung sieht in der Regel eine Drucküberwachung an der venösen Leitung bzw. an dem venösen Zugang vor, wie zum Beispiel durch das System VAM von Fresenius Medical Care, was in Dialysemaschinen standardmäßig vorgesehen ist. Die Überwachung des venösen Druckverlaufs gilt jedoch als notorisch unsicher, unter anderem weil der Druck abhängig von einer Vielzahl von Faktoren stark variieren kann. Daher wird eine VND zum Teil nur verzögert erkannt, weshalb der Patient bereits bis zum Auslösen des Alarms eine signifikante Menge Blut verloren haben kann. Ferner kommt es vor, dass beispielsweise aufgrund von Strömungswiderständen an der Nadel oder deren Befestigungsmaterial sowie bei unvollständigem Lösen der Nadel der Druckabfall so gering ist, dass dieser nicht als VND erkannt und gar kein Alarm ausgelöst wird.

Eine weitere Lösung besteht in der Erkennung einer Leckage an dem Zugang durch dort angebrachte Leckage- oder Feuchtigkeitssensoren, welche einen Blutverlust in die Umgebung überwachen, wie zum Beispiel durch VenAcc von Fresenius Medical Care und Redsense von Redsense Medical, welche ein Einweg-Sensorpad aufweisen, das zusammen mit der Nadel aufgeklebt wird und bereits geringe Mengen austretender Flüssigkeiten erkennt. Allerdings sind diese Vorrichtungen mit zum Teil erheblichen zusätzlichen Kosten verbunden, da hierfür teure Zusatzgeräte und Einwegteile benötigt werden, wobei menschliche Fehler nicht ausgeschlossen werden können.

Die bekannten Lösungen ermöglichen somit keine hundertprozentig sichere Erkennung von VND. Da außerdem durch Fehlanwendung bzw. Fehlbedienung oder zu eng gesetzte Grenzwerte die Wahrscheinlichkeit für Fehlalarme hoch ist, werden venöse Alarme häufig einfach weggedrückt, sodass trotz Anschlagen eines Alarms eine VND ggf. nicht rechtzeitig erkannt wird. Im Übrigen sind derartige bekannte Systeme recht aufwändig, da (Druck-/Feuchtigkeits-)Sensoren angebracht werden müssen, deren Signale in der Maschinen-eigenen Steuerung verarbeitet werden, wodurch die Maschine bzw. das extrakorporale Blutleitungssystem als Einweg-System verteuert wird.

Ferner ist aus JP 2016-214293 A eine Bilderfassungseinrichtung einer Dialyseeinrichtung bekannt, welche an den Nadeln oder Patientenzugängen angeordnete Marker einzeln überwacht und deren Position jeweils mit einer Vergleichsposition, z.B. einer zugehörigen Punktionsstelle, vergleicht, um ggf. eine Blutpumpe zu stoppen. Vorzugsweise wird dabei nur eine der Nadeln mit Markern versehen, da mehrere Marker bei der Bildverarbeitung leicht verwechselbar sind.

WO 2014/049357 A1 offenbart eine Sensorvorrichtung mit einem Sensorelement, das fest an einer Kanüle anbringbar ist und einen Einsteckabschnitt aufweist, und mit einer Sensoreinheit, welche eine Aufnahme zum Aufnehmen des Einsteckabschnitts sowie elektronische Messmittel zur Erfassung einer Verschiebung der Aufnahme relativ zu dem Einsteckabschnitt aufweist. Im Falle einer solchen Verschiebung wird ein Alarm ausgelöst, ein Ventil betätigt oder eine Pumpe ausgeschaltet.

Darüber hinaus betrifft WO 2009/042261 A1 eine Überwachungseinrichtung für einen Zugang zu einem Patientengefäß hinsichtlich Blutverlust während einer extrakorporalen Behandlung. Die Überwachungseinrichtung weist eine Bandage mit einem Polymer auf, das expandiert, wenn es feucht wird, und dadurch einen elektrischen Kontakt unterbricht oder ein Sensor, der ein Target überwacht, dieses nicht mehr erfassen kann. In einem solchen Fall wird die Therapie unterbrochen oder ein Alarm ausgelöst.

Aus US 2006/013059 A1 ist bekannt, eine venöse Nadeldislokation während einer Dialysebehandlung zu erfassen, indem ein Fotosensor mit einer venösen Nadel verbunden und durch eine nicht an der Nadel angebrachte lichtundurchlässige Abdeckung verdeckt wird, sodass bei einer Nadeldislokation der Fotosensor unter der Abdeckung hervorrutscht, wodurch Licht darauf fällt und ein Signal erzeugt. Anschließend wird ein Alarm ausgelöst und/oder die Gerätschaften ausgeschaltet werden.

### Kurzbeschreibung der Erfindung

Die Aufgabe der vorliegenden Erfindung besteht folglich darin, ein extrakorporales Blutleitungssystem mit (System-eigener) Sicherungseinrichtung insbesondere im Fall von VND zur Verfügung zu stellen, welches u.a. die vorstehend genannten Nachteile bestehender Systeme/Sicherungseinrichtungen behebt und insbesondere eine zuverlässige, schnelle Erkennung beispielsweise von VND sowie eine möglichst schnelle, zuverlässige Unterbrechung/Reduktion des Blutflusses ermöglicht. Darüber hinaus sollte das extrakorporale Blutleitungssystem bzw. dessen Sicherungseinrichtung gegen Blutverlust beispielsweise im Fall von VND möglichst einfach und kostengünstig sein.

Diese Aufgabe wird durch eine Sicherungseinrichtung eines extrakorporalen Blutleitungssystems bzw. durch ein extrakorporales Blutleitungssystem einer/für eine extrakorporale(n) Blutbehandlungsmaschine mit den Merkmalen des Patentanspruchs 1 gelöst. Vorteilhafte Ausgestaltungen der Erfindung sind Gegenstand der Unteransprüche.

Zum besseren Verständnis sei an dieser Stelle darauf hingewiesen, dass nachfolgend die Begriffe "stromaufwärts" und "stromabwärts" verwendet werden, was stets in Bezug auf eine Blutflussrichtung zu betrachten ist.

Die Aufgabe wird insbesondere gelöst durch eine erfindungsgemäße Sicherungseinrichtung mit einem Nadeldislokation-Erfassungs- und Absperrmechanismus eines extrakorporalen Blutleitungssystems für eine/einer extrakorporale(n) Blutbehandlungsmaschine, vorzugsweise Dialysemaschine, zum Auslösen einer Notabsperrung des extrakorporalen Blutleitungssystems der extrakorporalen Blutbehandlungsmaschine im Fall einer Nadeldislokation (insbesondere VDN). Die Sicherungseinrichtung weist einen Absperrmechanismus (z.B. Schlauchklemme oder Schließventil) auf, welcher dazu angepasst (konfiguriert) ist, einen Blutfluss durch eine Leitung des extrakorporalen Blutleitungssystems der/für die Blutbehandlungsmaschine zu reduzieren oder abzusperren. Ferner weist die Sicherungseinrichtung eine (rein mechanisch arbeitende/funktionierende) Betätigungs-/Auslöseeinrichtung (auch als Nadeldislokations-Erfassungseinrichtung bezeichnet) auf, welche dazu angepasst (konfiguriert) ist, eine Änderung einer vorbestimmten Ausgangs-Relativposition eines venösen Patientenzugangs/-anschlusses, vorzugsweise eine Zugangskanüle, und eines arteriellen Patientenzugangs/- anschlusses, vorzugsweise eine Zugangskanüle, des extrakorporalen Blutleitungssystems zueinander, welche die vorbestimmte Ausgangs-Relativposition zueinander haben oder auf diese eingestellt sind, zu erfassen und den Absperrmechanismus (8) (automatisch/selbsttätig) dann zu betätigen/auszulösen, wenn der venöse Patientenzugang/-anschluss (10) und der arterielle Patientenzugang/- anschluss (6) sich aus dieser vorbestimmten Ausgangs-Relativposition um einen vorbestimmten oder vorbestimmbaren Änderungsbetrag herausbewegen oder herausbewegt werden.

Die Betätigungs-/Auslöseeinrichtung der erfindungsgemäßen Sicherheitseinrichtung übernimmt somit prinzipiell zwei Funktionen, nämlich das Erfassen (Ansprechen auf) einer Änderung der ursprünglich eingestellten/vorliegenden Relativlage zwischen den beiden Patientenzugängen der extrakorporalen Blutleitungssystems und das Auslösen des zumindest einen Absperrmechanismus bei Erreichen/Überschreiten eines bestimmten/vorbestimmbaren Änderungsbetrags.

Die erfindungsgemäße Sicherungseinrichtung kann bei jeder extrakorporalen Blutbehandlung, insbesondere bei Dialysebehandlungen wie Hämodialyse, Hämofiltration und Hämodiafiltration, eingesetzt werden, bei welcher zumindest zwei Patientenzugänge/-anschlüsse, vorzugsweise Zugangskanülen, benötigt werden.

Der Vorteil einer solchen, neuartigen Sicherungseinrichtung gegenüber den bekannten Systemen besteht darin, dass nicht mehr Druckverluste/Druckänderungen innerhalb des extrakorporalen Blutleitungssystems erfasst werden müssen, die indirekt auf eine Nadel-Dislokation schließen lassen, sondern dass eine Positions- bzw. Relativlageänderung der beiden (venösen und arteriellen) Patientenanschlüsse des extrakorporalen Blutleitungssystems direkt erfasst werden und so unmittelbar eine Notabsperrung des extrakorporalen Blutleitungssystems schnell und zuverlässig bewirkt wird.

Grundsätzlich lassen sich zwei Varianten der erfindungsgemäßen Sicherungseinrichtung unterscheiden. Diese sind zum einen
- eine mechanische Kopplung der beiden Patientenzugänge, durch welche der Absperrmechanismus auslösbar ist, und zum anderen
- eine sensorische Überwachung der Relativposition der beiden Patientenzugänge sowie eine elektronische Ansteuerung des Absperrmechanismus.

Jede dieser Varianten kann unterschiedlich ausgeführt oder modifiziert werden. Diese Varianten und einige Ausführungsformen werden nachfolgend genauer beschrieben.

Die vorliegende Erfindung nutzt die technische Notwendigkeit, dass bei einer extrakorporalen Blutbehandlung mehrere, insbesondere zwei, Patientenzugänge gelegt werden, um die extrakorporale Blutbehandlungsmaschine (das extrakorporale Blutleitungssystem) an einen Blutkreislauf bzw. ein Blutgefäß des Patienten anzuschließen, welche an einer Patientenoberfläche wie Haut vorzugsweise klebend fixiert werden und somit in ihrer Position relativ zu dem Patienten sowie relativ zueinander festgelegt sind, also sich in ihrer Ausgangs-Relativposition befinden. Daraus folgt, dass bei einer Nadeldislokation von einem der Patientenzugänge der entsprechende Patientenzugang oder sogar beide Patientenzugänge sich bewegen und folglich eine Verschiebung der beiden Patientenzugänge relativ zueinander stattfindet, welche detektierbar/erfassbar ist. Entsprechend wird nach der vorliegenden Erfindung der Absperrmechanismus abhängig von der Relativposition ausgelöst/betätigt.

Ferner kann das Drucküberwachungssystem, welches standardmäßig in extrakorporalen Blutbehandlungsmaschinen (extrakorporalen Blutleitungssystemen) verbaut ist, von der erfindungsgemäßen Erfassungseinrichtung ausgelöst werden, wodurch ein maschinen-eigener Absperrmechanismus betätigt wird und/oder die erfindungsgemäße Sicherungseinrichtung besitzt ihren eigenen Absperrmechanismus (zusätzlich zu dem maschinen-eigenen Absperrmechanismus). Ein Absperren des Blutflusses durch die entsprechende Leitung auf einer venösen Seite des extrakorporalen Blutleitungssystems, also z.B. stromabwärts eines Dialysefilters einer Dialysemaschine, insbesondere an dem venösen Patientenzugang, durch den Maschinen-eigenen oder Sicherungseinrichtungs-eigenen Absperrmechanismus führt zu einem erhöhten Blutdruck stromaufwärts der Stelle, an welcher der Blutfluss abgesperrt ist. Ein Absperren des Blutflusses durch die entsprechende Leitung auf einer arteriellen Seite des extrakorporalen Blutleitungssystems, also z.B. stromaufwärts eines Dialysefilters einer Dialysemaschine, insbesondere an dem arteriellen Patientenzugang, durch den Maschinen-eigenen oder Sicherungseinrichtungs-eigenen Absperrmechanismus führt zu einem abfallenden Blutdruck stromabwärts der Stelle, an welcher der Blutfluss abgesperrt ist. In jedem Fall führt die Absperrung dazu, dass eine Blutpumpe der extrakorporalen Blutbehandlungsmaschine kein Blut aus dem Patientengefäß in die Umgebung pumpen kann.

Die vorstehend beschriebenen Druckänderungen erfolgen schnell und sind groß genug, dass sie sicher von dem in dem Behandlungssystem, insbesondere der Dialysemaschine, standardmäßig vorgesehenen Drucküberwachungssystem erfasst werden. Damit kann eine Reaktion der Blutbehandlungsmaschine eingeleitet werden, d.h. ein Alarm kann unmittelbar ausgegeben und ggf. die Blutpumpe abgeschaltet/angehalten werden. Vorteilhaft ist hierbei insbesondere, dass ein Alarm zwar manuell weggedrückt werden kann, dieser aufgrund der weiter anhaltenden Sperrung oder Reduzierung des Blutflusses jedoch sofort wieder anschlagen würde. Grundsätzlich kann der Absperrmechanismus der Sicherungseinrichtung an jeder Stelle des extrakorporalen Blutleitungssystems vorgesehen sein und ist nicht auf den venösen oder arteriellen Zugang beschränkt.

Der Absperrmechanismus der Sicherungseinrichtung kann aus einer oder mehreren, räumlich voneinander getrennten Baugruppen (z.B. Ventile, Schlauchklemmen, etc.) bestehen. Ferner kann er dazu angepasst sein, den Blutfluss durch das extrakorporale Blutleitungssystem gezielt an einer vorbestimmten Stelle abzusperren oder zu reduzieren, oder er kann dazu angepasst sein, den Blutfluss zufällig an einer von mehreren vorbestimmten Stellen abzusperren oder zu reduzieren.

Nach einem bevorzugten Aspekt der vorliegenden Erfindung ist die vorbestimmte Ausgangs-Relativposition des venösen Patientenzugangs/-anschlusses und des arteriellen Patientenzugangs/-anschlusses mechanisch durch die Erfassungseinrichtung, insbesondere Betätigungs-/Auslöseeinrichtung eingestellt bzw. festgelegt oder ist durch diese einstellbar bzw. festlegbar. In anderen Worten werden der venöse Patientenzugang und der arterielle Patientenzugang nach diesem Aspekt der Erfindung während einer extrakorporalen Blutbehandlung in ihrer Ausgangs-Relativposition mechanisch miteinander gekoppelt (beispielsweise über eine Verbindungsstange oder einen Gelenkmechanismus, der/die die Erfassungseinrichtung bildet). Dies entspricht der ersten der vorstehend genannten, grundsätzlichen Variante der erfindungsgemäßen Erfassungseinrichtung.

Es wird hierbei von einer extrakorporalen Blutbehandlung mit einem Doppelnadel-Patientenanschluss, vorzugsweise einer Doppelnadeldialyse, ausgegangen, bei welcher die beiden Patientenzugänge beispielsweise in ein arterielles und venöses Blutgefäß des Patienten eingeführt sind. Dadurch liegen die beinen Anschlüsse/Nadeln/Kanülen nahe beieinander und sind ferner entlang der Blutgefäße angeordnet, deren Verlauf in dem entsprechenden Abschnitt im weitesten Sinne linear bzw. parallel anzunehmen ist. Entsprechend kann davon ausgegangen werden, dass auch die Patientenzugänge in ihrer Ausgangs-Relativposition näherungsweise linear bzw. parallel zueinander liegen. Hierdurch wird das Herstellen einer mechanischen Kopplung beispielsweise durch eine Verbindungsstange/ein Verbindungsgestänge ermöglicht oder zumindest erleichtert.

Vorteilhafterweise können bei der Erfassungseinrichtung nach diesem Aspekt der Erfindung sehr einfache Bauteile verwendet werden, die beispielsweise durch

Spritzgießen günstig fertigbar sind. Aufgrund der Einfachheit der Erfassungseinrichtung ist deren Handhabung einfach und ein Potential für Fehlbedienungen durch das medizinische Personal ist gering. Darüber hinaus geben sich aufgrund der mechanischen Kopplung die beiden Patientenzugänge eine gegenseitige Zugentlastung, sodass bei einem leichten Zug oder Ruck, welcher ohne eine derartige Kopplung möglicherweise zu einer Nadeldislokation führen würde, ein Herausrutschen des Patientenzugangs ggf. verhindert wird.

Nach einem weiteren bevorzugten Aspekt der vorliegenden Erfindung weist die Erfassungseinrichtung bzw. Betätigungs-/Auslöseeinrichtung einen vorzugsweise stabförmigen Verbindungsabschnitt und zumindest einen vorzugsweise zapfenförmigen Betätigungs-/Auslöseabschnitt auf. Der Betätigungs-/Auslöseabschnitt ist mit dem vorzugsweise an dem venösen Patientenzugang/-anschluss und/oder vorzugsweise dem arteriellen Patientenzugang/-anschluss angebrachten Absperrmechanismus der Sicherungseinrichtung gekoppelt oder koppelbar. Ferner ist der Betätigungs-/Auslöseabschnitt dazu angepasst, den Absperrmechanismus zu betätigen/auszulösen.

Die bevorzugte Anbringung des Absperrmechanismus an dem venösen und/oder arteriellen Patientenzugang ist derart zu verstehen, dass der Absperrmechanismus oder ein Teil des Absperrmechanismus in diesem bevorzugten Fall unmittelbar an dem entsprechenden Patientenzugang angeordnet ist oder an der an den entsprechenden Patientenzugang angeschlossenen (Blut-) Leitung unmittelbar neben dem Patientenzugang angeschlossen ist oder als Anschlussstück zwischen dem Patientenzugang und der Leitung vorgesehen ist.

Grundsätzlich kann der mechanische Verbindungsabschnitt der Erfassungseinrichtung eine beliebige, geeignete Form haben, beispielsweise eine gekrümmte oder plattenförmige Verbindung. Gleichermaßen kann auch der Betätigungs-/Auslöseabschnitt der Sicherungseinrichtung eine beliebige Form haben, welche dazu angepasst ist, den Absperrmechanismus der Sicherungseinrichtung zu betätigen oder auszulösen. Vorzugsweise ist der zumindest eine Betätigungs-/Auslöseabschnitt zapfenförmig ausgebildet (wie vorstehend bereits angedeutet) und dazu angepasst, in ein Einsteckloch oder Öse des Absperrmechanismus eingesteckt zu werden, um diesen zu betätigen bzw. um den Blutfluss durch die entsprechende Leitung zu entsperren. Dabei ist die Länge des Zapfens derart dimensioniert, dass der Zapfen zum einen lang genug ist, sodass er sicher in den Absperrmechanismus einsteckbar ist und nicht einfach herausfallen kann, und zum anderen kurz genug ist, dass der Zapfen sich bei einer Nadeldislokation bei einem der Patientenzugänge aus dem Absperrmechanismus löst, bevor die über den Verbindungsabschnitt übertragene Zugkraft dazu führt, dass auch der andere der Patientenzugänge aus dem Patientengefäß rutscht.

Der Verbindungsabschnitt darf vorzugsweise geringfügig elastisch verformbar sein. Auf diese Weise ist es möglich, geringfügige Abweichungen der Lage der Patientenzugänge relativ zueinander auszugleichen. Allerdings darf die Elastizität nicht so groß sein, dass im Falle einer Nadeldislokation lediglich der Verbindungsabschnitt verformt wird, die Kopplung zwischen dem Absperrmechanismus und der Betätigungs-/Auslöseeinrichtung sich aber nicht löst.

Nach einem weiteren bevorzugten Aspekt der Erfindung ist der Absperrmechanismus, beispielsweise in Form einer in Schließrichtung federvorgespannten Schlauchklemme (nur schematisch in den Figuren dargestellt), in einer Offen-Stellung gehalten, wenn der vorzugsweise zapfenartige Betätigungs-/Auslöseabschnitt mit dem Absperrmechanismus gekoppelt ist, und in einer Absperr-Stellung überführt, wenn der Betätigungs-/Auslöseabschnitt von dem Absperrmechanismus getrennt ist. Somit ist gewährleistet, dass Patientenblut nur dann durch das extrakorporale Blutleitungssystem fließen kann, wenn die Betätigungs-/Auslöseeinrichtung am Absperrmechanismus angeschlossen ist. Es ist also nicht möglich, eine extrakorporale Blutbehandlung zu beginnen, ohne dass die Betätigungs-/Auslöseeinrichtung am Absperrmechanismus angebracht ist, oder in anderen Worten, die Absperrvorrichtung aktiviert ist.

Nach einem weiteren bevorzugten Aspekt der Erfindung ist der zumindest eine Betätigungs-/Auslöseabschnitt der Betätigungs-/Auslöseeinrichtung entlang einer Längsachse des Verbindungsabschnitts der Erfassungseinrichtung verschiebbar und sicherbar. Dabei kann die Verschiebbarkeit beispielsweise über eine Schiene entlang des Verbindungsabschnitts gewährleistet sein und kann der zumindest eine Betätigungs-/Auslöseabschnitt in seiner Position entlang des Verbindungsabschnittes beispielsweise über eine Klemme oder eine Schraube gesichert oder festgesetzt werden. Dies hat den Vorteil, dass der Abstand der Betätigungs-/Auslöseabschnitte zueinander stufenlos einstellbar ist.

Alternativ oder zusätzlich ist bevorzugt eine Anzahl von Betätigungs-/Auslöseabschnitten entlang des Verbindungsabschnitts, vorzugsweise in gleichen Abständen zueinander, angeordnet. Falls der Verbindungsabschnitt stabförmig ausgebildet ist und die Betätigungs-/Auslöseabschnitte zapfenförmig ausgebildet sind, hat die Betätigungs-/Auslöseeinrichtung also eine kammartige Form. Diese ist besonders kostengünstig herzustellen und im Gegensatz zu der vorstehend beschriebenen Ausführung mit verschiebbaren Betätigungs-/Auslöseabschnitten ist kein zusätzlicher Handgriff nötig, um diese zu sichern und um die Absperrvorrichtung funktionsbereit zu machen. Folglich ist das Potential für Fehlbedienung minimiert.

Nach einem weiteren bevorzugten Aspekt der Erfindung hat der Absperrmechanismus mehrere voneinander separate Teil-Absperrmechanismen, welche getrennt voneinander betätigbar/auslösbar sind. Dabei ist ein arterieller Teil-Absperrmechanismus an dem arteriellen Patientenzugang/-anschluss angebracht und ist vorzugsweise einstückig mit diesem gefertigt. Ferner ist ein venöser Teil-Absperrmechanismus an dem venösen Patientenzugang/-anschluss angebracht und ist vorzugsweise einstückig mit diesem gefertigt.

In anderen Worten kann jeder der beiden Teil-Absperrmechanismen dazu angepasst (konfiguriert) sein, den Blutfluss durch die entsprechende Leitung abzusperren oder zu reduzieren. Dabei ist nicht festgelegt, welcher der beiden Teil-Absperrmechanismen die Absperrung oder Reduzierung des Blutflusses auslöst. Dies ist lediglich davon abhängig, an welchem der beiden Teil-Absperrmechanismen die Betätigungsvorrichtung sich zuerst löst und dadurch die Absperrung durch den entsprechenden Teil-Absperrmechanismus auslöst. Die Betätigungs-/Auslöseeinrichtung ist hierbei ein loses Bauteil, welches mit beiden Teil-Absperrmechanismen gleichermaßen koppelbar ist. Darüber hinaus sind die beiden Patientenzugänge im Wesentlichen gleich ausgebildete Bauteile und jeder Patientenzugang ist mit einem der Teil-Absperrmechanismus vorzugsweise einstückig ausgebildet. Dies erhöht die zu fertigende Stückzahl und senkt somit die Fertigungskosten.

Nach einem weiteren bevorzugten Aspekt der Erfindung bildet die Betätigungs-/Auslöseeinrichtung (Erfassungseinrichtung) eine erste Betätigungs-/Auslöseeinrichtung, die dazu angepasst (konfiguriert) ist, den venösen Teil-Absperrmechanismus zu betätigen/auszulösen. Hierfür ist die erste Betätigungs-/Auslöseeinrichtung mit dem venösen Teil-Absperrmechanismus mechanisch gekoppelt oder koppelbar und ist mit dem arteriellen Patientenzugang/-anschluss oder dem arteriellen Teil-Absperrmechanismus gelenkig oder fest verbunden, vorzugsweise einstückig mit diesem ausgebildet. Darüber hinaus bildet die erfindungsgemäße Betätigungs-/Auslöseeinrichtung (Erfassungseinrichtung) eine zweite Betätigungs-/Auslöseeinrichtung, die dazu angepasst (konfiguriert) ist, den arteriellen Teil-Absperrmechanismus zu betätigen/auszulösen. Zu diesem Zweck ist diese mit dem arteriellen Teil-Absperrmechanismus mechanisch gekoppelt oder koppelbar und ist mit dem venösen Patientenzugang/-anschluss oder dem venösen Teil-Absperrmechanismus gelenkig oder fest verbunden, vorzugsweise einstückig mit diesem ausgebildet. D.h. dass in diesem bevorzugten Fall zwei Stangen oder Gestänge parallel zueinander angeordnet sind und jeweils einen der beiden Absperrmechanismen auslösen.

Bei einer Nadeldislokation, beispielsweise auf der venösen Seite des extrakorporalen Blutleitungssystems, verschiebt sich der venöse Patientenzugang zusammen mit dem venösen Teil-Absperrmechanismus relativ zu dem arteriellen Patientenzugang und dem daran angebrachten arteriellen Teil-Absperrmechanismus. Dadurch wird beispielsweise die erste Betätigungs-/Auslöseeinrichtung von dem venösen Teil-Absperrmechanismus entkoppelt, wodurch dieser ausgelöst/betätigt wird und der Blutfluss durch die diesem zugehörige Leitung abgesperrt wird. Gleichzeitig wird ggf. auch die zweite Betätigungs-/Auslöseeinrichtung von dem arteriellen Teil-Absperrmechanismus entkoppelt, wodurch dieser ggf. ausgelöst/betätigt wird und der Blutfluss durch die diesem zugehörige Leitung abgesperrt wird und umgekehrt.

Dabei sei darauf hingewiesen, dass es bereits ausreicht, wenn nur ein Teil-Absperrmechanismus ausgelöst wird. Insofern würde es prinzipiell auch ausreichen, nur einen der beiden Patientenanschlüsse/Patientenzugänge mit einem entsprechenden Absperrmechanismus zu versehen, wohingegen die Betätigungs-/Auslöseeinrichtung am anderen Patientenanschluss/Patientenzugang nur angelenkt/montiert ist (ohne Absperrmechanismus).

Die erste Betätigungs-/Auslöseeinrichtung ist beispielsweise an dem arteriellen Teil-Absperrmechanismus oder an dem arteriellen Patientenzugang gehalten oder fest mit diesem verbunden. Gleichermaßen ist die zweite Betätigungs-/Auslöseeinrichtung beispielsweise an dem venösen Teil-Absperrmechanismus oder an dem venösen Patientenzugang gehalten oder fest mit diesem verbunden. An jedem Patientenzugang oder an dem daran angeordneten Teil-Absperrmechanismus kann beispielsweise eine Öse oder Rille vorgesehen sein, an welcher eine der Betätigungs-/Auslöseeinrichtungen eingehängt bzw. einhängbar oder angeclipt bzw. anclipbar ist. Diese ist vorzugsweise kammartig oder als eine Art Zahnstange ausgebildet. In diesem Fall ist die Handhabung beim Koppeln der Betätigungs-/Auslöseeinrichtung mit der jeweiligen Teil-Absperreinrichtung einfach. Es muss lediglich gewährleistet sein, dass durch die Steifigkeit der Verbindung der Abstand zwischen den beiden Patientenanschlüssen im Wesentlichen festgelegt ist. Alternativ kann der Betätigungs-/Auslöseabschnitt einstückig mit dem entsprechenden Patientenzugang und/oder mit dem daran angebrachten Teil-Absperrmechanismus verbunden bzw. angespritzt sein. In diesem Fall ist die Absperrvorrichtung einfacher und günstiger zu fertigen. Somit sind beide Teil-Absperrmechanismen ggf. zusammen mit den zugehörigen Patientenzugängen gleiche Bauteile oder-gruppen und die zu fertigende Stückzahl kann erhöht werden. Ferner weist die Absperrvorrichtung in diesem Fall keine separaten, losen Teile auf, welche verloren oder fallen gelassen werden können.

Grundsätzlich kann dieses System auch derart variiert werden, dass nur eine Betätigungs-/Auslöseeinrichtung vorgesehen ist (wie dies vorstehend bereits angedeutet wurde), welche mit einem der Patientenzugänge wie vorstehend beschrieben verbunden ist und welche den nur an dem anderen der Patientenzugänge vorgesehenen Absperrmechanismus betätigt.

Nach einem anderen bevorzugten Aspekt der Erfindung ist die Betätigungs-/Auslöseeinrichtung dazu angepasst (konfiguriert), die Ausgangs-Relativposition des venösen Patientenzugangs/-anschlusses und des arteriellen Patientenzugangs/- anschlusses zueinander sensorisch oder optisch zu überwachen. Ferner ist die Betätigungs-/Auslöseeinrichtung dazu angepasst (konfiguriert), den Absperrmechanismus elektronisch, vorzugsweise kabellos, anzusteuern.

Dies entspricht der zweiten der vorstehend genannten, grundsätzlichen Variante der erfindungsgemäßen Absperrvorrichtung.

Wie auch bei der ersten Variante kann die Absperrvorrichtung nach der zweiten Variante derart ausgelegt sein, dass der Absperrmechanismus den Blutfluss durch die entsprechende Leitung absperrt oder reduziert, solange die Absperrvorrichtung bzw. die Betätigungs-/Auslöseeinrichtung nicht aktiviert wurde, z.B. durch einen Knopfdruck oder Schalter. Somit ist gewährleistet, dass keine extrakorporale Blutbehandlung starten kann, ohne dass die Absperrvorrichtung einsatzbereit ist.

Nach diesem Aspekt der Erfindung ist es vorteilhaft, dass der Absperrmechanismus an jeder geeigneten Stelle des extrakorporalen Blutleitungssystems angebracht sein kann, wie zum Beispiel unmittelbar stromaufwärts der Blutpumpe der extrakorporalen Blutbehandlungsmaschine. Dies ermöglicht eine größere Flexibilität bei der Konstruktion und die Zugangskanülen sind leichter. Ebenso ist es gegenüber der Variante mit einer mechanischen Kopplung nicht möglich, die Absperrung durch Hängenbleiben an der Betätigungs-/Auslöseeinrichtung fehlauszulösen.

Die Datenverbindung kann beispielsweise über Funk, Bluetooth oder Kabel bereitgestellt werden. Das Datenverarbeitungsmodul kann als separates Gerät vorgesehen sein, in der extrakorporalen Blutbehandlungsmaschine selbst verbaut sein oder an einem beliebigen, geeigneten Ort vorgesehen sein. Für die Positionsüberwachung können an den Patientenzugängen Marker oder Sensoren angebracht werden. Ferner kann für die Positionsüberwachung prinzipiell jeder geeignete Positions- und/oder Abstandssensor verwendet werden, wie beispielsweise Infrarot- oder induktive Sensoren. Weitere Beispiele sind GPS-Überwachung, Ultraschall- oder Lasersensoren oder die Positionserkennung über eine Kamera und zugehörige Bildverarbeitung.

Grundsätzlich ist die Sicherungseinrichtung nach diesem Aspekt der Erfindung für jede beliebige Anordnung von Patientenzugängen denkbar, wobei beispielsweise bei an verschiedenen Patientenarmen angebrachten Patientenzugängen ggf. weitere Referenzpunkte/Marker an der Patientenhaut neben dem entsprechenden Zugang gesetzt werden müssen, um zu vermeiden, dass eine Armbewegung des Patienten die Absperrung auslöst. Hieraus ist ersichtlich, dass die Absperrvorrichtung nach diesem Aspekt der Erfindung auch für andere Einsätze geeignet ist, bei welchen nur ein Patientenzugang gelegt wird, wie z.B. bei einer Infusion, wo eine erfindungsgemäße Absperrvorrichtung ermöglicht, ein Auslaufen von Medikamenten etc. im Fall einer Nadeldislokation zu verhindern.

Nach einem weiteren bevorzugten Aspekt der Erfindung weist der Absperrmechanismus ein durch ein Federelement rückstellbares Leitungsklemmelement zum Abklemmen der Leitung der extrakorporalen Blutbehandlungsmaschine oder ein Sperrventil auf.

Das Sperrventil kann beispielsweise ein elektromagnetisch, -motorisch oder mechanisch angetriebenes Ventil sein. Dies ist entweder mechanisch ansteuerbar, indem beispielsweise durch einen Betätigungsabschnitt nach einem der vorstehenden Aspekte der Erfindung einen elektrischen Kontakt in dem Absperrmechanismus hergestellt wird, oder elektrisch ansteuerbar.

### Figurenbeschreibung

Die Erfindung wird im Folgenden anhand bevorzugter Ausführungsformen beschrieben, wobei Merkmale verschiedener Ausführungsformen untereinander ausgetauscht und/oder kombiniert werden können. Es versteht sich, dass Einzelheiten der beschriebenen bevorzugten Ausführungsformen die Erfindung als solche nicht beschränken und sich für den Fachmann naheliegend verschiedenartige Änderungen, Modifikationen und/oder Äquivalente ergeben können, die als solche allesamt im Rahmen des durch die beigefügten Ansprüche definierten Schutzbereichs der Erfindung liegen. Ferner werden in der nachfolgenden Beschreibung der bevorzugten Ausführungsformen gleiche Bezugszeichen für einander entsprechende Merkmale verwendet, welche ggf. nur einmal beschrieben werden, jedoch in jeder der vorgestellten Ausführungsformen vorgesehen sein können.
Fig. 1 zeigt eine erfindungsgemäße Sicherungseinrichtung nach einer ersten Ausführungsform der Erfindung während einer extrakorporalen Blutbehandlung;
Fig. 2 ist eine Seitenansicht und eine Draufsicht eines Verbindungselements der Sicherungseinrichtung nach der ersten Ausführungsform;
Fig. 3 ist eine Detailansicht eines Patientenzugangs/-anschlusses mit einer erfindungsgemäßen Sicherungseinrichtung nach der ersten Ausführungsform;
Fig. 4 zeigt verschiedene Anordnungen bzw. Ausgangs-Relativpositionen von Patientenzugängen/-anschlüssen der Sicherungseinrichtung nach der ersten Ausführungsform;
Fig. 5 zeigt eine erfindungsgemäße Sicherungseinrichtung nach einer zweiten Ausführungsform der Erfindung,
Fig. 6 ist eine schematische Darstellung einer erfindungsgemäßen Sicherungseinrichtung nach einer dritten Ausführungsform der Erfindung während einer extrakorporalen Blutbehandlung.

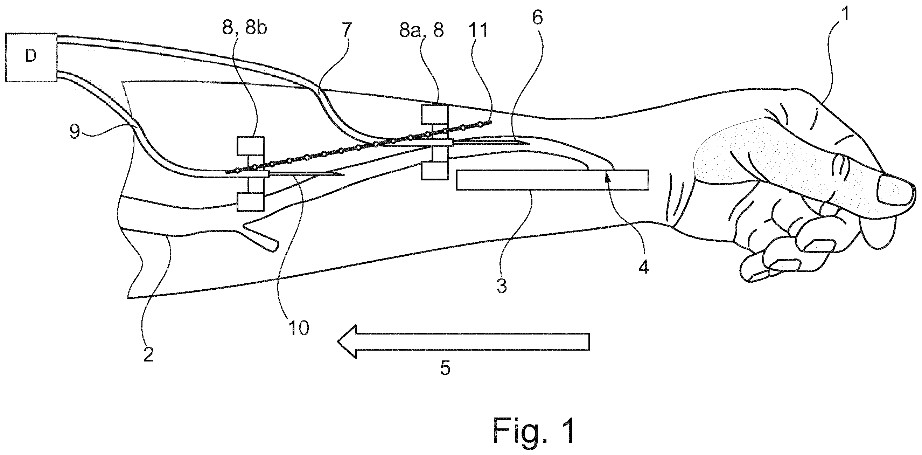

Fig. 1 zeigt beispielhaft einen Patientenarm 1 während einer extrakorporalen Blutbehandlung, wobei von einer Doppelnadeldialyse ausgegangen wird, welche die nachfolgend beschriebene Anordnung voraussetzt. Eine Patientenvene 2 und eine Patientenarterie 3 sind durch einen Shunt 4 verbunden. Ein breiter Pfeil markiert eine Blutflussrichtung 5 durch die Patientenvene 2. Der Shunt 4 stellt sicher, dass eine extrakorporale Blutbehandlungsmaschine D an ein großvolumiges Patientenblutgefäß, insbesondere eine Patientenvene 2, mit einem ausreichend großen Durchfluss angeschlossen werden kann. In nachfolgenden Figuren und Ausführungsformen ist die extrakorporale Blutbehandlungsmaschine D nicht gesondert abgebildet, es versteht sich jedoch, dass diese bei jeder der beschriebenen Ausführungsformen vorgesehen ist.

Stromabwärts des Shunts 4 ist in der Blutflussrichtung 5 eine arterielle Zugangskanüle 6 als ein arterieller Patientenzugang/-anschluss in das Patientenblutgefäß 2 eingeführt. Die Zugangskanüle 6 ist mit einer arteriellen (Blut-) Leitung 7 eines extrakorporalen Blutleitungssystems 7, 9 der extrakorporalen Blutbehandlungsmaschine (nicht dargestellt) fluidverbunden, sodass Blut aus dem Patientenblutgefäß 2 über die arterielle Zugangskanüle 6 zu der extrakorporalen Blutbehandlungsmaschine geleitet wird.

An der arteriellen Leitung 7 ist ein arterieller Teil-Absperrmechanismus 8a, welcher ein Teil eines (Gesamt-)Absperrmechanismus 8 ist, in unmittelbarer Nähe (oder direkt an) der arteriellen Zugangskanüle 6 angeordnet, insbesondere an der Verbindungsstelle zwischen der arteriellen Leitung 7 und der arteriellen Zugangskanüle 6, an welcher diese beiden miteinander fluidverbunden sind. Der arterielle Teil-Absperrmechanismus 8a ist dazu angepasst, wenn er betätigt oder ausgelöst wird, einen Blutfluss durch die arterielle Leitung 7 abzusperren oder zu reduzieren.

Das aus dem Patientenblutgefäß 2 abgeleitete Blut durchfließt die extrakorporale Blutbehandlungsmaschine bzw. einen Filter (nicht weiter gezeigt) und wird über eine venöse (Blut-) Leitung 9 des extrakorporalen Blutleitungssystems sowie über eine mit der venösen Leitung 9 fluidverbundene venöse Zugangskanüle 10 zurück in das Patientenblutgefäß 2 geleitet. Dabei ist die venöse Zugangskanüle 10 an einer Seite stromabwärts der arteriellen Zugangskanüle 6 in der Blutflussrichtung 5 in das Patientenblutgefäß 2 eingeführt und dient als ein venöser Patientenzugang/-anschluss.

Ein venöser Teil-Absperrmechanismus 8b, welcher ein Teil des (Gesamt-) Absperrmechanismus 8 ist, ist an der venösen Leitung 9 in unmittelbarer Nähe (oder direkt an) der venösen Zugangskanüle 10 angeordnet, insbesondere an der Verbindungsstelle zwischen der venösen Leitung 9 und der venösen Zugangskanüle 10, an welcher diese beiden miteinander fluidverbunden sind. Der venöse Teil-Absperrmechanismus 8b ist dazu angepasst, wenn er betätigt oder ausgelöst wird, einen Blutfluss durch die venöse Leitung 8 zu unterbrechen oder zu reduzieren. Der arterielle und der venöse Teilabsperrmechanismus 8a, 8b bilden gemeinsam den Absperrmechanismus 8.

Die beiden Zugangskanülen 6, 10 sind in einer vorbestimmten Ausgangs-Relativposition zueinander angeordnet, wobei ein Abstand zwischen den beiden Zugangskanülen 6, 10 einen vorbestimmten, maximalen Abstand nicht überschreitet, auf welchen später genauer eingegangen wird. Die arterielle Zugangskanüle 6 und die venöse Zugangskanüle 10 sind jeweils an einer Patientenoberfläche, insbesondere Patientenhaut, in ihrer Ausgangs-Relativposition vorzugsweise klebend fixiert.

In dem in Fig. 1 dargestellten Zustand, in welchem die beiden Zugangskanülen (Anschlüsse) 6, 10 die vorbestimmte Ausgangs-Relativposition zueinander haben, ist die arterielle Zugangskanüle 6 bzw. die arterielle Leitung 7 über
- den arteriellen Teil-Absperrmechanismus 8a,
- eine kamm-, raster- oder zahnstangenartig ausgebildete Betätigungs-/Auslöseeinrichtung (Erfassungseinrichtung) 11 und
- den venösen Teil-Absperrmechanismus 8b
mechanisch mit der venösen Zugangskanüle 10 bzw. der venösen Leitung 9 gekoppelt.

Die Betätigungs-/Auslöseeinrichtung 11 wirkt derart mit den beiden Teil-Absperrmechanismen 8a, 8b zusammen, dass einerseits die Teil-Absperrmechanismen 8a, 8b in einer Offen-Stellung sind (gehalten werden), wenn diese mit der Betätigungs-/Auslöseeinrichtung 11 gekoppelt sind, und folglich der Blutfluss durch die jeweils zugehörige Leitung 7, 9 freigegeben ist und andererseits die Teil-Absperrmechanismen 8a, 8b in einer Absperr-Stellung sind (gehalten werden), in welcher der Blutfluss durch die zugehörige Leitung 7, 9 abgesperrt oder reduziert wird, wenn diese nicht mit der Betätigungs-/Auslöseeinrichtung 11 gekoppelt sind. Das heißt, wenn beispielsweise die Kopplung zwischen dem arteriellen Teil-Absperrmechanismus 8a und der Betätigungs-/Auslöseeinrichtung 11 sich löst oder gelöst wird, schaltet der arterielle Teil-Absperrmechanismus 8a von einer Offen-Stellung in eine Absperrstellung um und unterbricht oder reduziert somit den Blutfluss durch die arterielle Leitung 7. Gleiches gilt für eine Funktionsweise des venösen Teil-Absperrmechanismus 8b.

Dabei sei an dieser Stelle ausdrücklich darauf hingewiesen, dass die Anordnung nur eines Teil-Absperrmechanismus für die Funktionsfähigkeit der Sicherungseinrichtung ausreicht, wobei in diesem Fall die Betätigungs-/Auslöseeinrichtung 11 an einem Endabschnitt mit diesem einen Teil-Absperrmechanismus gekoppelt und an dem anderen Endabschnitt beispielsweise direkt an der anderen Zugangskanüle montiert ist. Auch besteht die Möglichkeit, zwei Betätigungs-/Auslöseeinrichtungen z.B. in Form von den dargestellten Stangen anzuordnen, die jeweils einen der beiden Teil-Absperrmechanismen (unabhängig voneinander) betätigen.

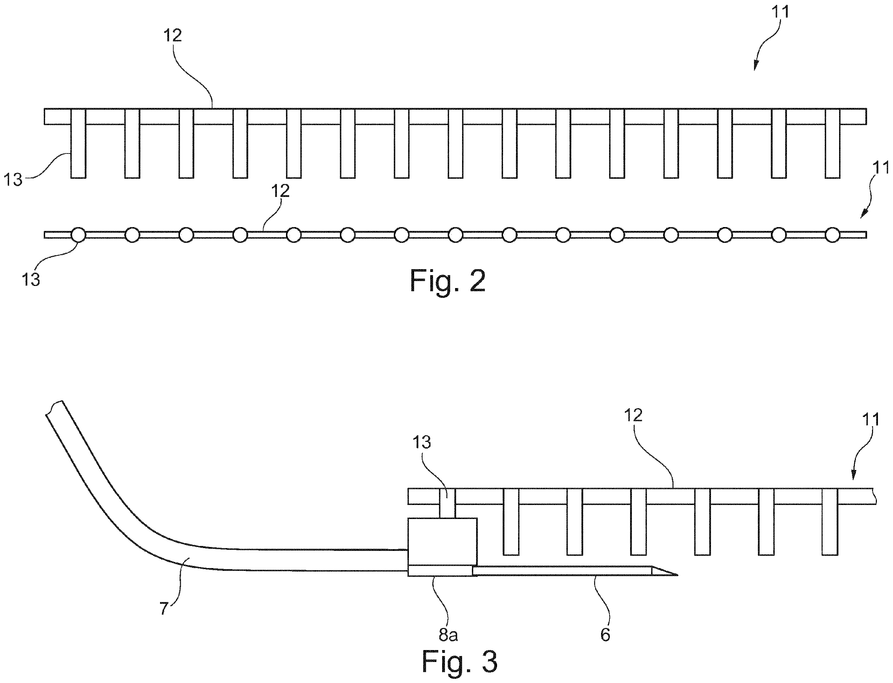

Die Betätigungs-/Auslöseeinrichtung 11 und deren Funktionsweise werden nachfolgend mit Bezug auf die Fig. 2 und Fig. 3 genauer beschrieben.

Fig. 2 zeigt die Betätigungs-/Auslöseeinrichtung 11 nach der ersten Ausführungsform, welche als Verbindungsschiene oder Stange mit kammartigen, bzw. axial beabstandeten Anschlussstellen ausgebildet ist. Die Betätigungs-/Auslöseeinrichtung 11 weist im konkreten Fall einen im Wesentlichen stabförmigen Verbindungsabschnitt (gerader Stab) 12 auf, welcher steif oder nur eingeschränkt elastisch in dessen Längsrichtung verformbar/dehnbar ist, sodass dessen Enden im Wesentlichen stets den gleichen Abstand zueinander haben. Die Länge des Verbindungsabschnittes 12 definiert den maximalen Abstand, welchen die beiden Teil-Absperrmechanismen 8a, 8b und folglich auch die beiden Zugangskanülen 6, 10 zueinander haben dürfen, wenn sie sich in der vorbestimmten Ausgangs-Relativposition zueinander befinden. Entlang des Verbindungsabschnittes 12 sind in gleichmäßigen Abständen Zapfen/Vorsprünge 13 mit vorzugsweise rundem Querschnitt als Betätigungs-/Auslöseabschnitte angeordnet, welche in einer Ebene liegen und welche mit dem Verbindungsabschnitt 12 einstückig, vorzugsweise als Spritzgussteil, ausgebildet sind.

Fig. 3 zeigt beispielhaft eine Detailansicht der arteriellen Zugangskanüle 6 mit der daran angeschlossenen arteriellen Leitung 7 und einem Teil der erfindungsgemäßen Sicherungseinrichtung nach der ersten Ausführungsform, genauer, dem arteriellen Teil-Absperrmechanismus 8a, welcher mit der Betätigungs-/Auslöseeinrichtung 11 gekoppelt ist. Diese Kopplung ist dadurch gewährleistet, dass einer der Zapfen/Vorsprünge 13 in ein an dem arteriellen Teil-Absperrmechanismus 8a vorgesehenes Einsteckloch eingesteckt ist, wodurch der arterielle Teil-Absperrmechanismus 8a in eine Offen-Stellung versetzt/gehalten ist. Beispielsweise drückt der Zapfen 13 entgegen einer Rückstell- oder Federkraft auf einen in das Einsteckloch hineinragenden Hebel oder Stift, welcher dazu vorgesehen ist, die Leitung 7 abzuklemmen bzw. den Blutfluss durch die Leitung 7 deutlich zu reduzieren, wenn der Zapfen 13 den Hebel oder Stift nicht beiseite drückt.

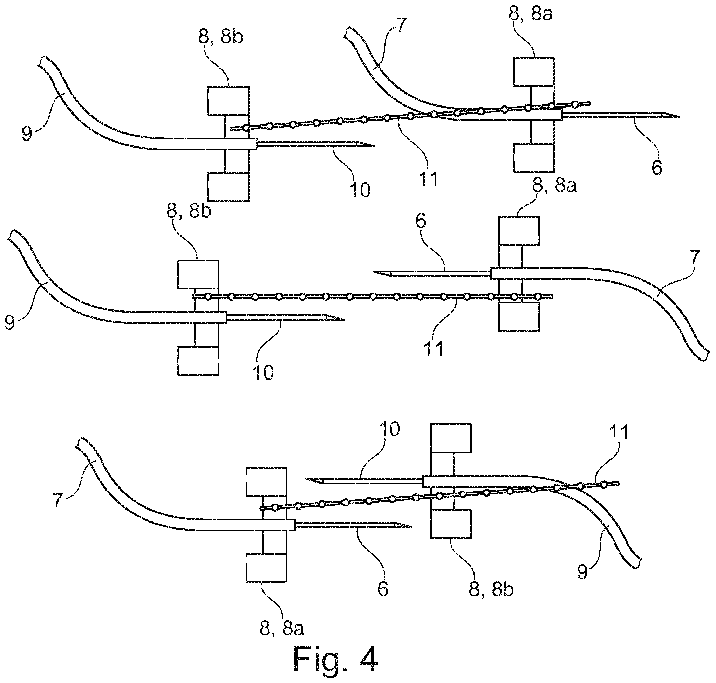

Fig. 4 zeigt verschiedene, beispielhafte Anordnungen bzw. Ausgangs-Relativpositionen der beiden Zugangskanülen 6, 10 zueinander nach der Absperrvorrichtung gemäß der ersten Ausführungsform.

In einer ersten Anordnung (oben) sind die beiden Zugangskanülen 6, 10 im Wesentlichen parallel zueinander ausgerichtet sind, in einer zweiten Anordnung (mittig) sind sie im Wesentlichen gegenläufig oder entgegengesetzt zueinander ausgerichtet und in einer dritten Anordnung (unten) ist der Abstand der beiden Zugangskanülen 6, 10 deutlich verringert und die Betätigungs-/Auslöseeinrichtung 11 ragt deutlich über eine der Teil-Absperrvorrichtungen 8a, 8b hinaus. Eine derart flexible Anordnung ist aufgrund des runden Querschnitts der Zapfen 13 sowie aufgrund der Anzahl von entlang des Verbindungsabschnitts 12 verteilten Zapfen 13 möglich. Dadurch kann die Betätigungs-/Auslöseeinrichtung 11 relativ zu jedem der Teil-Absperrmechanismen 8a, 8b um die Längsachse des in den entsprechenden Teil-Absperrmechanismus 8a, 8b eingesteckten Zapfen 13 verdreht werden. Ferner können zwei beliebige Zapfen 13 der Betätigungs-/Auslöseeinrichtung 11 in die Teil-Absperrmechanismen 8a, 8b eingesteckt werden, wodurch die Betätigungs-/Auslöseeinrichtung 11 an den Abstand der Teil-Absperrmechanismen 8a, 8b und folglich an deren vorbestimmte Ausgangs-Relativposition zueinander anpassbar ist.

Da die Abstände der Zapfen zueinander festgelegt sind, sind während des Einsatzes bei einer extrakorporalen Blutbehandlung hierdurch auch die Abstände der Teil-Absperrmechanismen 8a, 8b und damit der Zugangskanülen zueinander festgelegt, an denen die Teil-Absperrmechanismen montiert/ausgebildet sind. Kommt es beispielsweise aufgrund eines auf die venöse Leitung 9 wirkenden Rucks zu einer venösen Nadeldislokation, rutscht entsprechend die venöse Zugangskanüle 10 zumindest teilweise aus der Patientenvene 2 heraus, wodurch sich auch der zugehörige venöse Teil-Absperrmechanismus 8b bewegt. Das heißt, die beiden Zugangskanülen 6, 10 und die entsprechenden Teil-Absperrmechanismen 8a, 8b werden aus ihrer durch die Betätigungs-/Auslöseeinrichtung 11 vorbestimmten/vorbestimmbaren Ausgangs-Relativposition heraus zueinander verschoben und deren Abstand zueinander ändert sich. Da jedoch der Verbindungsabschnitt 12 der Betätigungs-/Auslöseeinrichtung 11 im Wesentlichen steif oder nur begrenzt elastisch verformbar ist, rutscht dabei zumindest aus einem der Teil-Absperrmechanismen 8a, 8b der in dessen Einsteckloch eingesteckte Zapfen 13 mit Erreichen/Überschreiten eines maximal zulässigen Positionsänderungsbetrags (bestimmt sich u.a. aus der Elastizität des Verbindungabschnitts 12 und dem Spiel zwischen Zapfen und Einsteckloch) heraus und betätigt somit den Absperrmechanismus 8, indem der entsprechende Teil-Absperrmechanismus 8a, 8b betätigt und in die Absperr-Stellung geschaltet wird. Die Länge der Zapfen ist bevorzugt derart ausgelegt, dass der Zapfen sicher aus dem Einsteckloch eines der Teil-Absperrmechanismen 8a, 8b rutscht, bevor der von der venösen Zugangskanüle 10 über die Betätigungs-/Auslöseeinrichtung 11 auf die arterielle Zugangskanüle 6 übertragene Ruck auch zu einer arteriellen Nadeldislokation führen kann.

Insofern lässt ich die Betätigungs-/Auslöseeinrichtung 11 auch als Erfassungseinrichtung bezeichnen, da diese im Ansprechen auf eine Änderung der Relativlage der beiden Zugangskanülen zumindest einen Teil-Absperrmechanismus auslöst und somit das gesamte extrakorporale Blutleitungssystem notsperrt.

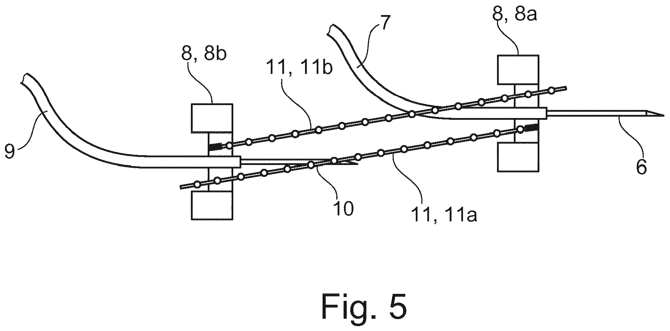

Fig. 5 zeigt schematisch eine erfindungsgemäße Sicherungseinrichtung nach einer zweiten Ausführungsform. Diese weist eine erste Betätigungs-/Auslöseeinrichtung 11a auf, welche fest mit dem arteriellen Teil-Absperrmechanismus 8a verbunden bzw. spritzgegossen (schematisch durch schwarzen Block dargestellt) und mit dem venösen Teil-Absperrmechanismus 8b gekoppelt ist, um diesen ggf. zu betätigen. Ferner ist eine entsprechende zweite Betätigungs-/Auslöseeinrichtung 11 b vorgesehen, welche fest mit dem venösen Teil-Absperrmechanismus 8b verbunden bzw. spritzgegossen und mit dem arteriellen Teil-Absperrmechanismus 8a gekoppelt ist, um diesen ggf. zu betätigen. Somit können die Patientenzugänge mitsamt den daran angeordneten oder befestigten Komponenten der Sicherungseinrichtung als Gleichteile ausgebildet werden.

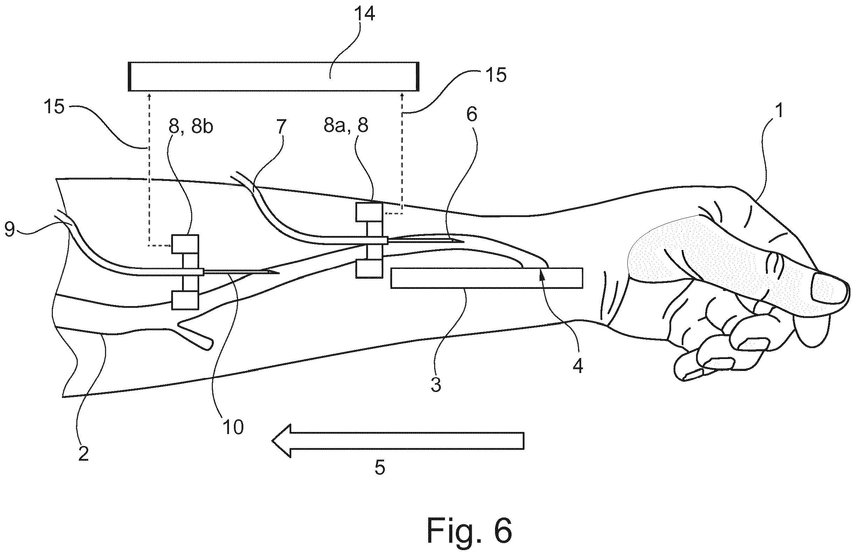

Fig. 6 zeigt schematisch eine erfindungsgemäße Sicherungseinrichtung nach einer dritten Ausführungsform der Erfindung während einer extrakorporalen Blutbehandlung. Im Wesentlichen unterscheidet diese sich von der ersten Ausführungsform darin, dass die beiden Teil-Absperrmechanismen 8a, 8b nicht mechanisch durch die Betätigungs-/Auslöseeinrichtung 11 gekoppelt sind und der Absperrmechanismus 8 bzw. die Teil-Absperrmechanismen 8a, 8b elektrisch betätigbar oder auslösbar sind. Die Betätigungs-/Auslöseeinrichtung 11 nach der dritten Ausführungsform weist ein Datenverarbeitungsmodul 14 als einen Betätigungs-/Auslöseabschnitt auf, wobei während einer extrakorporalen Blutbehandlung eine Datenverbindung 15 als ein Verbindungsabschnitt zwischen dem Datenverarbeitungsmodul 14 und den Teil-Absperrmechanismen 8a, 8b hergestellt ist. An den Teil-Absperrmechanismen 8a, 8b sind beispielsweise Marker oder Sensoren angebracht, mittels derer das Datenverarbeitungsmodul 14 die Position der Teil-Absperrmechanismen 8a, 8b zueinander überwachen und deren Bewegung aus einer vorbestimmten Ausgangs-Relativposition heraus während einer extrakorporalen Blutbehandlung erkennen kann. Das Datenverarbeitungsmodul 14 ist dazu angepasst, wenn eine derartige Bewegung erkannt wird, über die Datenverbindung 15 den Absperrmechanismus 8 bzw. die Teil-Absperrmechanismen 8a, 8b anzusteuern, um diese von einer Offen-Stellung in eine Absperr-Stellung zu schalten.

### Liste der Referenzzeichen

- D: Dialysemaschine
- 1: Patientenarm
- 2: Patientenvene
- 3: Patientenarterie
- 4: Shunt
- 5: Blutflussrichtung
- 6: arterieller Patientenzugang/-anschluss (arterielle Zugangskanüle)
- 7: arterielle (Blut-) Leitung
- 8: Absperrmechanismus
- 8a: arterieller Teil-Absperrmechanismus
- 8b: venöser Teil-Absperrmechanismus
- 9: venöse (Blut-) Leitung
- 10: venöser Patientenzugang/-anschluss (venöse Zugangskanüle)
- 11: Betätigungs-/Auslöseeinrichtung/Erfassungseinrichtung
- 11 a: erste Betätigungs-/Auslöseeinrichtung/Erfassungseinrichtung
- 11b: zweite Betätigungs-/Auslöseeinrichtung/Erfassungseinrichtung
- 12: Verbindungsabschnitt
- 13: Betätigungs-/Auslöseabschnitte (Zapfen)
- 14: Betätigungs-/Auslöseabschnitte (Datenverarbeitungsmodul)
- 15: Verbindungsabschnitt (Datenverbindung)

## Patentansprüche

1. Sicherungseinrichtung eines extrakorporalen Blutleitungssystems für eine extrakorporale Blutbehandlungsmaschine, vorzugsweise Dialysemaschine, zum Auslösen einer Notabsperrung des extrakorporalen Blutleitungssystems im Fall einer Nadeldislokation, mit
einem Absperrmechanismus (8), welcher dazu angepasst ist, einen Blutfluss durch eine Leitung (7, 9) der extrakorporalen Blutbehandlungsmaschine zu reduzieren oder abzusperren,
**gekennzeichnet durch** eine Betätigungs-/Auslöseeinrichtung (11), welche dazu angepasst ist, eine Änderung einer vorbestimmten Ausgangs-Relativposition eines venösen Patientenzugangs/-anschlusses (10), vorzugsweise Zugangskanüle, und eines arteriellen Patientenzugangs/-anschlusses (6), vorzugsweise Zugangskanüle, des extrakorporalen Blutleitungssystems zueinander, welche die vorbestimmte Ausgangs-Relativposition zueinander haben oder auf diese eingestellt sind, zu erfassen und den Absperrmechanismus (8) dann zu betätigen/auszulösen, wenn der venöse Patientenzugang/-anschluss (10) und der arterielle Patientenzugang/-anschluss (6) sich aus dieser vorbestimmten Ausgangs-Relativposition um einen vorbestimmten oder vorbestimmbaren Änderungsbetrag herausbewegen oder herausbewegt werden.

2. Sicherungseinrichtung nach Anspruch 1, wobei die Betätigungs-/Auslöseeinrichtung (11) einen vorzugsweise stabförmigen Verbindungsabschnitt (12) und zumindest einen vorzugsweise zapfenförmigen Betätigungs-/Auslöseabschnitt (13) hat, welcher mit dem an dem venösen Patientenzugang/-anschluss (10) und/oder dem arteriellen Patientenzugang/-anschluss (6) angebrachten Absperrmechanismus (8) gekoppelt oder koppelbar ist und dazu angepasst ist, den Absperrmechanismus (8) zu betätigen/auszulösen.

3. Sicherungseinrichtung nach Anspruch 2, wobei der Absperrmechanismus (8) in einer Offen-Stellung ist, wenn der Betätigungs-/Auslöseabschnitt (13) mit dem Absperrmechanismus gekoppelt ist, und in einer Absperr-Stellung ist, wenn der Betätigungs-/Auslöseabschnitt (13) von dem Absperrmechanismus (8) getrennt ist.

4. Sicherungseinrichtung nach Anspruch 2 oder 3, wobei der zumindest eine Betätigungs-/Auslöseabschnitt (13) der Betätigungs-/Auslöseeinrichtung (11) entlang einer Längsachse des Verbindungsabschnitts (12) verschiebbar und sicherbar ist oder eine Anzahl von Betätigungs-/Auslöseabschnitten (13) entlang des Verbindungsabschnitts (12) angeordnet ist.

5. Sicherungseinrichtung nach einem der Ansprüche 1 bis 4, wobei
der Absperrmechanismus (8) mehrere voneinander separate Teil-Absperrmechanismen (8a, 8b) hat, welche getrennt voneinander betätigbar/auslösbar sind, und
ein arterieller Teil-Absperrmechanismus (8a) an dem arteriellen Patientenzugang/-anschluss (6) angebracht und vorzugsweise einstückig mit diesem gefertigt ist und ein venöser Teil-Absperrmechanismus (8b) an dem venösen Patientenzugang/-anschluss (10) angebracht ist und vorzugsweise einstückig mit diesem gefertigt ist.

6. Sicherungseinrichtung nach Anspruch 5, wobei
die Betätigungs-/Auslöseeinrichtung eine erste Betätigungs-/Auslöseeinrichtung (11a) ist und die Absperrvorrichtung ferner eine zweite Betätigungs-/Auslöseeinrichtung (11b) aufweist,
die erste Betätigungs-/Auslöseeinrichtung (11a) mit dem venösen Teil-Absperrmechanismus (8b) gekoppelt oder koppelbar ist, dazu angepasst ist, diesen zu betätigen/auszulösen, und mit dem arteriellen Patientenzugang/-anschluss (6) und/oder dem arteriellen Teil-Absperrmechanismus (8a) verbunden, vorzugsweise einstückig mit diesem ausgebildet ist, und
die zweite Betätigungs-/Auslöseeinrichtung (11b) mit dem arteriellen Teil-Absperrmechanismus (8a) gekoppelt oder koppelbar ist, dazu angepasst ist, diesen zu betätigen/auszulösen, und mit dem venösen Patientenzugang/-anschluss (10) und/oder dem venösen Teil-Absperrmechanismus (8b) verbunden, vorzugsweise einstückig mit diesem ausgebildet ist.

7. Sicherungseinrichtung nach Anspruch 1, wobei die Betätigungs-/Auslöseeinrichtung (11) dazu angepasst ist, die Ausgangs-Relativposition des venösen Patientenzugangs/-anschlusses (10) und des arteriellen Patientenzugangs/- anschlusses (6) zueinander sensorisch zu überwachen und/oder den Absperrmechanismus (8) elektronisch, vorzugsweise kabellos, anzusteuern.

8. Sicherungseinrichtung nach einem der vorstehenden Ansprüche, wobei der Absperrmechanismus (8) ein durch ein Federelement rückstellbares Leitungsklemmelement zum Abklemmen der Leitung (7, 9) der extrakorporalen Blutbehandlungsmaschine oder ein Sperrventil aufweist.

## Claims

1. A safety device of an extracorporeal blood conduit system for an extracorporeal blood treatment machine, preferably dialysis machine, for triggering emergency shut-off of the extracorporeal blood conduit system in the event of a needle dislocation, the safety device comprising
a shut-off mechanism (8) which is adapted to reduce or shut-off blood flow through a conduit (7, 9) of the extracorporeal blood treatment machine,
**characterized by** an actuating/triggering unit (11) which is adapted to monitor a change in a predetermined relative starting position of a venous patient access/connection (10), preferably access cannula, and an arterial patient access/connection (6), preferably access cannula, of the extracorporeal blood conduit system with respect to each other, the venous patient access/connection (10) and the arterial patient access/connection (6) having said predetermined relative starting position relative to each other or being adjusted thereto, and to actuate/trigger the shut-off mechanism (8) when the venous patient access/connection (10) and the arterial patient access/connection (6) move out of said predetermined relative starting position by a predetermined or predeterminable changing amount.

2. The safety device according to claim 1, wherein the actuating/triggering unit (11) includes a preferably rod-shaped connecting portion (12) and at least one pivot-shaped actuating/triggering portion (13) which is coupled or coupleable to the shut-off mechanism (8) arranged on the venous patient access/connection (10) and/or on the arterial patient access/connection (6) and is adapted to actuate/trigger the shut-off mechanism (8).

3. The safety device according to claim 2, wherein the shut-off mechanism (8) is in an open position when the actuating/triggering portion (13) is coupled to the shut-off mechanism and is in a shut-off position when the actuating/triggering portion (13) is disconnected from the shut-off mechanism (8).

4. The safety device according to claim 2 or 3, wherein the at least one actuating/triggering portion (13) of the actuating/triggering unit (11) can be displaced and secured along a longitudinal axis of the connecting portion (12) or a number of actuating/triggering portions (13) is arranged along the connecting portion (12).

5. The safety device according to one of claims 1 to 4, wherein
the shut-off mechanism (8) includes a plurality of partial shut-off mechanisms (8a, 8b) separate from each other which can be actuated/triggered separately from each other, and
an arterial partial shut-off mechanism (8a) is arranged at the arterial patient access/connection (6) and is preferably formed in one piece therewith and a venous partial shut-off mechanism (8b) is arranged at the venous patient access/connection (10) and is preferably formed in one piece with therewith.

6. The safety device according to claim 5, wherein
the actuating/triggering unit is a first actuating/triggering unit (11a) and the shut-off mechanism further includes a second actuating/triggering unit (11b),
the first actuating/triggering unit (11a) is or can be coupled to the venous partial shut-off mechanism (8b), is adapted to actuate/trigger the latter and is connected to, preferably formed in one piece with, the arterial patient access/connection (6) and/or the arterial partial shut-off mechanism (8a), and
the second actuating/triggering unit (11b) is coupled or coupleable to the arterial partial shut-off mechanism (8a), is adapted to actuate/trigger the latter and is connected to, preferably formed in one piece with, the venous patient access/connection (10) and/or the venous partial shut-off mechanism (8b).

7. The safety device according to claim 1, wherein the actuating/triggering unit (11) is adapted to monitor the predetermined relative starting position of the venous patient access/connection (10) and of the arterial patient access/connection (6) relative to each other by sensors and/or to electronically, preferably in a wireless manner, control the shut-off mechanism (8).

8. The safety device according to one of the preceding claims, wherein the shut-off mechanism (8) includes a conduit clamping element adapted to be reset by a spring element for clamping the conduit (7, 9) of the extracorporeal blood treatment machine or a stop valve.

## Revendications

1. Dispositif de sécurité d'un système de conduite de sang extracorporel pour une machine de traitement de sang extracorporel, de préférence une machine de dialyse, pour déclencher une fermeture d'urgence du système de conduite de sang extracorporelle en cas de dislocation d'une aiguille, avec
un mécanisme d'arrêt (8), lequel est adapté pour réduire ou arrêter un écoulement de sang à travers un conduit (7, 9) de la machine de traitement de sang extracorporelle,
**caractérisé par** un dispositif d'actionnement-déclenchement (11), lequel est adapté pour détecter un changement d'une position relative de sortie prédéterminée d'un accès/raccord veineux du patient (10), de préférence une canule d'accès, et d'un accès/raccord artériel du patient (6), de préférence une canule d'accès, du système de circulation sanguine extracorporelle, l'un par rapport à l'autre, qui présentent la position relative de sortie prédéterminée l'un par rapport à l'autre ou sont réglés sur celle-ci, et pour ensuite actionner/déclencher le mécanisme d'arrêt (8) lorsque l'accès/raccord veineux du patient (10) et l'accès/raccord artériel du patient (6) se déplacent ou sont déplacés hors de ladite position relative de sortie prédéterminée d'une quantité de changement prédéterminée ou pouvant être prédéterminée.

2. Dispositif de sécurité selon la revendication 1, dans lequel le dispositif d'actionnement/déclenchement (11) présente une section de connexion (12), de préférence en forme de tige, et au moins une section d'actionnement/déclenchement (13), de préférence en forme de broche, laquelle est couplée ou peut être couplée au mécanisme d'arrêt (8) fixé à l'accès/raccord veineux du patient (10) et/ou à l'accès/raccord artériel du patient (6) et est adaptée pour actionner/déclencher le mécanisme d'arrêt (8).

3. Dispositif de sécurité selon la revendication 2, dans lequel le mécanisme d'arrêt (8) est dans une position ouverte lorsque la section d'actionnement-déclenchement (13) est couplée au mécanisme d'arrêt, et est dans une position fermée lorsque la section d'actionnement-déclenchement (13) est séparée du mécanisme d'arrêt (8).

4. Dispositif de sécurité selon la revendication 2 ou 3, dans lequel la au moins une section d'actionnement/déclenchement (13) du dispositif d'actionnement/déclenchement (11) peut être déplacée et fixée le long d'un axe longitudinal de la section de connexion (12) ou un certain nombre de sections d'actionnement/déclenchement (13) sont agencées le long de la section de connexion (12).

5. Dispositif de sécurité selon l'une quelconque des revendications 1 à 4, dans lequel
le mécanisme d'arrêt (8) présente plusieurs mécanismes d'arrêt partiels (8a, 8b), lesquels sont séparés les uns des autres et peuvent être actionnés ou déclenchés séparément, et
un mécanisme d'arrêt partiel artériel (8a) est monté sur l'accès/raccord artériel du patient (6) et est de préférence fabriqué d'un seul tenant avec celui-ci, et un mécanisme d'arrêt partiel veineux (8b) est monté sur l'entrée/raccord veineux du patient (10) et est de préférence fabriqué d'un seul tenant avec celui-ci.

6. Dispositif de sécurité selon la revendication 5, dans lequel
le dispositif d'actionnement-déclenchement est un premier dispositif d'actionnement-déclenchement (11a) et le dispositif d'arrêt présente en outre un second dispositif d'actionnement-déclenchement (11b),
le premier dispositif d'actionnement-déclenchement (11a) est couplé ou peut être couplé au mécanisme d'arrêt partiel veineux (8b), est adapté pour actionner/déclencher celui-ci, et est connecté, de préférence formé d'un seul tenant avec l'accès/raccord artériel du patient (6) et/ou le mécanisme d'arrêt partiel artériel (8a), et
le second dispositif d'actionnement-déclenchement (11b) est couplé ou peut être couplé au mécanisme d'arrêt partiel artériel (8a), est adapté pour actionner/déclencher celui-ci, et est connecté à, de préférence formé d'un seul tenant avec, l'accès/raccord veineux du patient (10) et/ou le mécanisme d'arrêt partiel veineux (8b).

7. Dispositif de sécurité selon la revendication 1, dans lequel le dispositif d'actionnement/déclenchement (11) est adapté pour surveiller par capteur la position relative de sortie de l'accès/raccord veineux (10) du patient et de l'accès/raccord artériel (6) du patient l'un par rapport à l'autre et/ou pour commander électroniquement, de préférence sans fil, le mécanisme d'arrêt (8).

8. Dispositif de sécurité selon l'une quelconque des revendications précédentes, dans lequel le mécanisme d'arrêt (8) présente un élément de serrage de conduite pouvant être rappelé par un élément à ressort pour le serrage de la conduite (7, 9) de la machine de traitement de sang extracorporel ou une soupape d'arrêt.
